Europäisches Patentamt

European Patent Office

Office européen des brevets

(11) Publication number: **0 219 522**
**B1**

# EUROPEAN PATENT SPECIFICATION

(45) Date of publication of patent specification: **26.09.90**

(21) Application number: **86902457.0**

(22) Date of filing: **18.04.86**

(86) International application number:
**PCT/GB86/00217**

(87) International publication number:
**WO 86/05970 23.10.86 Gazette 86/23**

(51) Int. Cl.⁵: **A 61 F 13/00, A 61 L 15/16**

(54) WOUND DRESSINGS.

(30) Priority: **18.04.85 GB 8509977**

(43) Date of publication of application:
**29.04.87 Bulletin 87/18**

(45) Publication of the grant of the patent:
**26.09.90 Bulletin 90/39**

(84) Designated Contracting States:
**DE FR GB IT SE**

(56) References cited:
**EP-A-0 053 936**
**EP-A-0 099 758**
**FR-A-2 380 688**
**GB-A-2 092 006**
**GB-A-2 127 389**
**US-A-3 903 882**

(73) Proprietor: **Charcoal Cloth Ltd.**
**East Wing Bridgewater Lodge 160 Bridge Road**
**Maidenhead, Berkshire SL6 8DG (GB)**
(73) Proprietor: **Juhasz, Laszlo**
**18 Clovelly Avenue**
**London NW9 (GB)**

(72) Inventor: **JUHASZ, Laszlo**
**18 Clovelly Avenue**
**London NW9 (GB)**
Inventor: **McLEOD, Angus, Ian**
**Raplea Farthing Green Lane**
**Stoke Poges Buckinghamshire (GB)**

(74) Representative: **Perry, Robert Edward et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to anti-bacterial wound dressings. In particular, it relates to integral dressings which can be used to cover contaminated, discharging malodorous wounds, and assist in their treatment. More specifically, it relates to wound dressings comprising activated carbon.

The utility of carbonised fabric in surgical dressings has been appreciated for over 50 years. GB—A—0,386,867 discloses surgical dressings comprising woven or entangled carbonised fibres. Such dressings are also disclosed as supports for therapeutic or antiseptic materials and it is stated that "the dressings will hold in considerable quantities iodine, formol, lime, oxygen, bacillary toxins, and the like". The use of, say, iodine, in such dressings appears to be a consequence of the adsorptive characteristics of charcoal cloths.

GB—A—1,301,101 discloses a particularly useful, and commercially used, process for preparing activated carbon products in fibrous form. Rayon, for example, is impregnated with a solution of inorganic halides and then activated in a controlled heating step. The products, i.e. activated carbon cloth or felts, adsorb both organic materials and bacteria.

Surgical dressings using activated charcoal impregnated with anti-bacterial agent, within an envelope of permeable material, are disclosed in EP—A—0,053,936; the adsorptive sites of the activated charcoal are no more than 20% saturated with an anti-microbial agent, preferably iodine.

A disadvantage of such a dressing is that the agent incorporated in the dressing inherently limits the bacteria-adsorbing characteristics of the charcoal and could adversely affect wound healing. Further, the charcoal cloth can easily fragment, and carbon particles can find their way into the wound.

EP—A—0,099,758 discloses a three-layered composite (but not integral) wound dressing comprising a semi-permeable membrane, a permeable supporting and reinforcing layer, and a non-stick, self-sealing biodegradable tissue interface. The permeable layer may be an activated carbon cloth.

GB—A—2,127,389 discloses a surgical dressing comprising activated charcoal cloth or felt which has been produced so that it contains elemental silver distributed throughout. Such a product is at least bacteriostatic, but may not "fix" bacteria or facilitate wound healing.

It is an object of the invention to provide an anti-bacterial wound dressing which has an integrated structure and assists wound healing. In other words, the wound dressing should provide a barrier against bacterial contamination and mechanical injury, and also provide controlled water vapour transmission and controlled heat loss.

An integral anti-bacterial wound dressing according to the present invention comprises four layers which are, in order,
(1) a first layer of a permeable material.
(2) a layer of a semi-permeable material;
(3) a layer of charcoal fabric; and
(4) a second layer of a permeable material;
in which layers 1, 2 and 4 are substantially co-extensive and surround the charcoal fabric layer (3), and are bound together in the surrounding area; and in which the semi-permeable material (2) is adhesive, whereby layer 1 is bound to layer 3 and, around layer 3, to layer 4.

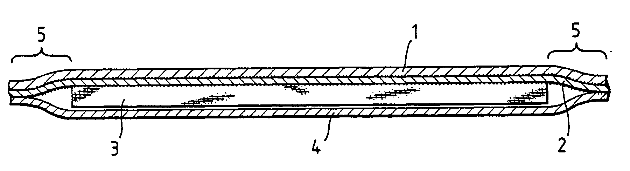

The accompanying drawing is an enlarged cross-sectional side view (not to scale) of a wound dressing which is an illustrative embodiment of the present invention.

The drawing shows a first layer of permeable material 1, a layer of a semi-permeable material 2, an activated charcoal fabric 3 and a second layer of permeable material 4. The permeable layers 1 and 4 and the semi-permeable layer are bonded at the border area 5 of the product, i.e. around the fabric 3.

It is intended that layer 4 should come into contact with a wound. In this position, bacteria in the atmosphere which come into contact with layers 1 and 2 are prevented from passing to the wound.

Owing to the adhesive nature of the semi-permeable material, one entire surface of the charcoal fabric is bonded to the first layer of permeable material. The two layers of permeable material are bound together, via the semi-permeable material, in the area which borders the charcoal fabric. The only area of non-adherence between pairs of adjacent layers in the dressing (shown clearly in the drawing) is over the adjacent faces of the charcoal fabric and layer 4; the opposite face of layer 4 is entirely suitable as that intended to contact the wound, in use. If desired, the double-sided adhesive properties are apparent only at elevated temperature, e.g. because the semi-permeable material is thermoplastic and can be made tacky, say, at 40 to 60°C, and may be induced by applying a heat-press.

A wound dressing of the invention may carry a marker indicating the opposite side to the wound-facing surface.

The "enveloping" layers may be of different or, often, the same permeable material. Examples of suitable materials are natural or synthetic rubber, nylon, polyester, polyurethane and rayon acetate, and other suitable synthetic polymers. The material should be in the form of a fabric or film having a pore size of, say, 50 to 500 µm, e.g. about 150 to 200 µm. The wound-facing layer may instead be biodegradable, e.g. of a collagen or a collagen-alginate material.

The charcoal fabric is, for example, a cloth or felt of the activated type, e.g. prepared as described in GB—A—1,301,101. It is preferably a woven, knitted or non-woven fabric of activated carbon, but any activated charcoal fabric, made from, e.g. paper or other cellulosic material, may be used. For ease of handling, the charcoal cloth may be laminated to a substrate of any suitable material, e.g. a polyester viscose such as FBR 33 (available from BFF), but this is not critical.

The semi-permeable material may be thermoplastic, e.g. having a softening point of 70 to 120°C; suitable materials are polyamides such as polycaprolactam and other "nylons", and also polycarbonates. Further, inherently adhesive semi-permeable materials are known, e.g. in the form of a "transfer tape". A double-sided transfer tape, having a pore size of less than 50 µm, derived from rayon acetate and polyurethane, is available from DRG or the 3M Company Ltd. Alternatively, semi-permeable adhesive materal can be sprayed on to double release papers or, using a single release paper, on to the outer dressing layer.

Preferably, the semi-permeable material has a pore size of less than 20 µm. It should provide water vapour transmission of 200 to 2000 g/m²/24 h, for the dressing as a whole. The effective pore size of the dressing may be less than 2 µm.

The size of a wound dressing of the invention may be defined as desired. For example, the charcoal fabric may be about 140 × 90 mm and the other three layers each about 150 × 100 mm in area, so that the border around the charcoal fabric is about 10 mm wide. An alternative embodiment comprises a relatively wide border on two sides of the charcoal fabric, so that the product has more the appearance of a strip. Again, the dressing can be formulated as a bandage. For use, the dressing may be provided together with a release liner.

Depending on the intended use, a dressing of the invention may be required to have high liquid absorption capability. This can be achieved by including a layer of an absorbent material, e.g. cotton or a suitable foam. Such a layer is preferably positioned between the wound-facing layer and the charcoal fabric. The layer itself may be thin but the material may take up 10 times its own weight of liquid.

Products of the invention are of utility as field dressings. For this purpose, the product may be integrated with a bandage. For example, a waterproof cover and stretch bandage may be provided.

A product of the invention has anti-bacterial characteristics in that it adsorbs bacteria, reduces bacterial growth (by limiting oxygen availability), and provides a bacterial barrier, thereby minimising external and cross-contamination. The dressing has wound-healing characteristics because it controls water vapour transmission, thereby maintaining a humid environment which allows the natural wound-healing processes to function.

The wound-facing permeable layer is essentially non-adherent to the wound. The dressing can be absorbent with respect to exudate, and eliminate offensive odours.

A primary advantage of a wound dressing of the invention is that it is anti-bacterial and assists wound management. It can be used for the treatment of infected and discharging, ulcerated and permanent, cancerous and malodorous, and contaminated and burn wounds. Its structure is integrated. In particular, the charcoal fabric is bound over its area; fraying, which occurs if such a material is merely loosely held, and which potentially leads to carbon fabric particles being shed into a wound, is prevented.

Three examples of dressings of the invention have been prepared. Their respective sizes are 100 mm × 150 mm, 150 mm × 190 mm and 190 mm × 280 mm. Their respective dressing surface areas are 158 cm², 285 cm² and 532 cm². Their respective apparent surface areas are 1600 m², 2160 m² and 6720 m². Their respective weights are 3.8g, 7g and 14.4g. Their respective fluid contents on saturation with water are 12.5 ml, 25 ml and 50 ml. In each case, the fluid absorption on saturation is 375%, the fluid absorption rate is 100 mg/sec, the water vapour transmission is 1088 g/m²/24 h, the carbon particle release with respect to activated charcoal cloth is 0.01%, and the bacterial absorption (reduction in log.) is 3—5.

## Claims

1. An integral multi-layer anti-bacterial wound dressing which comprises, in order,
   a first layer (1) of a permeable material;
   a layer (3) of charcoal fabric; and
   a second layer (4) of a permeable material;
   in which the first layer (1) and the second layer (4) are substantially co-extensive and surround the layer (3) of charcoal fabric, and are bound together in the surrounding area;
   characterised by a layer (2) of an adhesive semi-permeable material between the first layer (1) and the layer (3) of charcoal fabric and substantially co-extensive with the first layer (1) and the second layer (4), its adhesiveness binding together the first layer (1) and the layer (3) of charcoal fabric, and in said surrounding area, the first layer (1) and the second layer (4).

2. A wound dressing according to claim 1, wherein the layer (3) of charcoal fabric is an activated carbon fabric.

3. A wound dressing according to claim 1 or claim 2, wherein the semi-permeable material has a pore size of less than 20 µm.

4. A wound dressing according to any preceding claim, characterised by water vapour transmission of 200 to 2000 g/m²/24h.

## Patentansprüche

1. Ein integraler, vielschichtiger, antibakterieller Wundverband, umfassend der Reihe nach:
   eine erste Schicht (1) aus einem durchlässigen Material;
   eine Schicht (3) aus Aktivkohlengewebe; und
   eine zweite Schicht (4) aus einem durchlässigen Material;
   worin die erste Schicht (1) und die zweite Schicht (4) im wesentlichen gleich ausgedehnt sind und die Schicht (3) aus Aktivkohlengewebe umgeben und in dem umgebenden Gebiet verbunden sind;

gekennzeichnet durch eine Schicht (2) aus einem haftenden halbdurchlässigen Material zwischen der ersten Schicht (1) und der Schicht (3) aus Aktivkohlengewebe und im wesentlichen gleich ausgedehnt wie die erste Schicht (1) und die zweite Schicht (4), wobei ihre Haftung die erste Schicht (1) und die Schicht (3) aus Aktivkohlengewebe und in dem umgebenden Gebiet die erste Schicht (1) und die zweite Schicht (4) zusammenbindet.

2. Wundverband nach Anspruch 1, worin die Schicht (3) aus Aktivkohlengewebe ein aktiviertes Carbongewebe ist.

3. Wundverband nach Anspruch 1 oder Anspruch 2, worin das halbdurchlässige Material eine Porengröße von weniger als 20 µm aufweist.

4. Wundverband nach einem der vorangehenden Ansprüche, gekennzeichnet durch eine Wasserdampfdurchlässigkeit von 200 bis 2000 g/m²/24 h.

## Revendications

1. Pansement antibactérien multicouche d'une seule pièce pour plaies qui comprend, dans l'ordre:

une première couche (1) en une matière perméable,

une couche (3) en tissu de charbon, et

une seconde couche (4) en une matière perméable, dans lequel la première couche (1) et la seconde couche (4) sont sensiblement de même étendue et entourent la couche (3) en tissu de charbon, et sont liées ensemble dans la zone périphérique, caractérisé par une couche (2) en une matière adhésive semi-perméable entre la première couche (1) et la couche (3) en tissu de charbon et sensiblement de même étendue que la première couche (1) et la seconde couche (4), et dont l'adhésivité lie ensemble la première couche (1) et la couche (3) en tissu de charbon, et dans ladite zone périphérique, la première couche (1) et la seconde couche (4).

2. Pansement pour plaies selon la revendication 1, dans lequel la couche (3) en tissu de charbon est un tissu de charbon actif.

3. Pansement pour plaies selon la revendication 1 ou la revendication 2, dans lequel la matière semi-perméable a une taille de pores inférieure à 20 µm.

4. Pansement pour plaies selon l'une des revendications précédentes, caractérisé par une transmission de vapeur d'eau de 200 à 2000 g/m²/24 h.